# EUROPEAN PATENT APPLICATION

(11) **EP 1 508 294 A1**
(43) Date of publication of application: **23.02.2005**
(21) Application number: 04019494.6
(22) Date of filing: 17.08.2004
(51) Int. Cl.: A61B 1/00

(54) **Endoscope hood**

(30) Priority: 21.08.2003 JP 2003297905; 06.07.2004 JP 2004199751
(71) Applicant: Olympus Corporation, Tokyo 151-0072 (JP)
(72) Inventor: Nozue, Kota, Kings Park New York 11754 (US)
(74) Representative: Schmidt, Steffen J., Dipl.-Ing.

(57) **Abstract**

An endoscope hood according to the present invention comprises: a hood portion provided to an insertion portion of an endoscope for being inserted into the body cavity, which includes a tube channel having a suctioning function, so as to extend in the forward viewing direction ahead of the distal end surface of an observation optical member for observing the tissue within the body cavity; a ridge formed on at least a part of the hood portion, which has a slope formed such that the cross-sectional thickness thereof becomes smaller toward the forward viewing direction. At least a part of the opening of the tube channel having the suctioning function is formed on the slope. The opening at the forward viewing direction is formed so as to be positioned closer to the observation window than the distal end of the hood portion formed near the tube channel.

## Description

### BACKGROUND OF THE INVENTION

### 1. Field of the Invention

The present invention relates to an endoscope hood integrally or detachably provided to the distal end of the insertion portion of an endoscope.

### 2. Description of the Related Art

An endoscope is principally formed of an insertion portion for being inserted into the body cavity, and an operation portion provided to the proximal end side of the insertion portion. Provided to the portion between the operation portion and the insertion portion are: an observation optical system for observing the object; an illumination optical system for illuminating the tissue which is to be observed; an air/water feed channel for providing air or water; a forceps channel for inserting a forceps; and so forth.

The insertion portion includes: an observation window; an illumination port; air/water feed opening; and forceps opening, on the distal end portion thereof. The observation optical system is provided to the proximal end of the observation window. The illumination optical system is provided to the proximal end of the illumination port. The air/water feed opening communicates with the air/water feed channel. On the other hand, the forceps opening communicates with the forceps channel.

On the other hand, the operation portion includes: an eyepiece optical system provided to the proximal end of the observation optical system; a forceps insertion opening for inserting the forceps into the forceps channel for treatment or the like; and so forth. Furthermore, the endoscope includes a universal cord extending from the operation portion. A light-guide optical-fiber bundle for providing illumination light to the illumination optical system, and the air/water feed channel, are inserted into the universal cord so as to extend from the proximal end of the operation portion therethrough.

With the endoscope employing the optical observing method, the observation optical system comprises: an objective lens provided to the observation window; an image-guide optical-fiber bundle for transmitting the optical object image taken in by the objective lens; an eyepiece lens provided so as to face the proximal end of the image-guide optical-fiber bundle; and so forth. On the other hand, with the endoscope employing the electronic imaging method, the observation optical system comprises: an objective lens provided to the observation window; and an image pickup apparatus including an image pickup device disposed at the focal position of the objective lens. In this case, the electric signals which are photoelectrically converted by the image pickup device are transmitted through a signal cable extending from the image pickup apparatus to an unshown camera control unit. The camera control unit creates video signals from the electric signals, and outputs the video signals thus created to a display device. Thus, an endoscopic image is displayed on a screen of the display device.

At the time of observing the body cavity with the endoscope, illumination light is cast onto the tissue which is to be observed, from a light source device through the illumination port. The optical image of the tissue within the body cavity which is to be observed, is taken in by the objective lens through the observation window. Thus, the physician can observe the optical image of the tissue through the eyepiece lens, or observe the endoscopic image displayed on the display device.

At the time of the physician performing treatment of a tissue portion within the body cavity while observing the tissue portion with the endoscope, the physician inserts an treatment tool or the like through the forceps channel for resection of the tissue, biopsy of the tissue, or the like. At the time of resection or biopsy, the physician can operate the endoscope so as to provide water or air to the body cavity through the air/water feed channel, or so as to perform suctioning of water, waste, or the like, through the forceps channel.

At the time of the physician performing treatment or the like while observing the body cavity with the endoscope, the endoscope must be held so as to maintain the suitable distance between the observation window and the tissue portion which is to be observed, for obtaining clear endoscopic images. Accordingly, the endoscope includes a hood on the distal end thereof for maintaining the suitable distance between the observation window and the tissue portion which is to be observed.

For example, with the endoscope disclosed in Japanese Examined Utility Model Registration Application Publication No. 59-15605, the endoscope includes a hood mounted on the outer surface of the distal end of the insertion portion, wherein the front end of the hood is in contact with the tissue which is to be observed during observation. This allows the physician to observe the moving tissue which is to be observed, such as mucous tissue or the like while maintaining the suitable distance between the tissue which is to be observed and the observation window.

On the other hand, with the endoscope disclosed in Japanese Unexamined Patent Application Publication No. 2000-197603, the distal end of the endoscope is formed in a dome shape, wherein the forceps-channel opening is formed around the apex thereof, and the observation window and illumination port are formed behind the forceps-channel opening, for facilitating operation for inserting the insertion portion of the endoscope into the body cavity.

However, with the endoscope hood disclosed in Japanese Examined Utility Model Registration Application Publication No. 59-15605, the endoscope hood is mounted so as to surround the outer circumference of the distal end portion of the insertion portion of the endoscope. This leads to the increased maximum diameter of the distal end portion of the insertion portion with the endoscope hood mounted thereon.

Furthermore, the endoscope hood mounted on the distal end portion may obstruct the field of view of the observation optical system, leading to shading. In order to prevent the aforementioned problem, the endoscope hood is formed with a suitable thickness, a suitable length extending from the distal end surface of the insertion portion up to the front surface of the endoscope hood, and so forth, giving consideration to the positional relation between: the observation window; the illumination ports; and the forceps opening, the viewing angle of the objective lens provided to the observation window, and so forth.

On the other hand, the endoscope disclosed in Japanese Unexamined Patent Application Publication No. 2000-197603 has a configuration wherein the forceps channel opening is formed at the front end of the distal end portion of the endoscope. This configuration may lead to a problem that the forceps channel opening directly comes in contact with the mucous tissue or the like within the body cavity at the time of the physician performing suctioning through the forceps channel opening at the time of observing the tissue, resulting in a problem of suctioning of the mucous tissue.

### SUMMARY OF THE INVENTION

It is a first object of the present invention to provide an endoscope hood which allows the physician to observe the tissue with a predetermined distance between the observation window provided on the insertion portion of the endoscope and the intended tissue portion while maintaining sufficient viewing angle of the observation optical system.

It is a second object of the present invention to provide an endoscope hood which allows the physician to operate the endoscope with the endoscope hood mounted thereon without a problem of suctioning of the mucous tissue, as well as maintaining the small diameter of the distal end portion of the insertion portion of the endoscope.

An endoscope hood according to the present invention comprises: a hood portion provided to an insertion portion of an endoscope for being inserted into the body cavity, which includes a tube channel having a suctioning function, so as to extend in the forward viewing direction ahead of the distal end surface of an observation optical member for observing the tissue within the body cavity; and a ridge formed on at least a part of the hood portion, which has a slope formed such that the cross-sectional thickness thereof becomes smaller toward the forward viewing direction;
wherein at least a part of the opening of the tube channel having the suctioning function is formed on the slope, and the opening is formed such that the front end thereof is positioned ahead of the ridge in the forward viewing direction.

The above and other objects, features and advantages of the invention will become more clearly understood from the following description referring to the accompanying drawings.

### BRIEF DESCRIPTION OF THE DRAWINGS

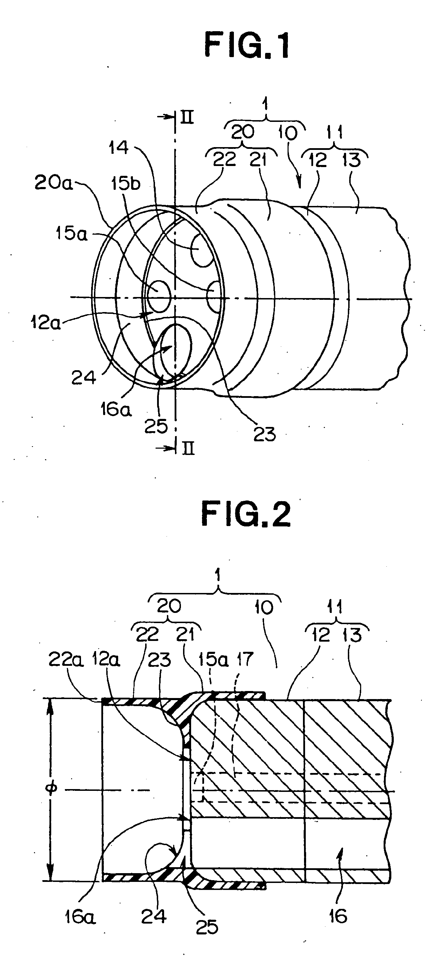

Figs. 1 and 2 are diagrams for describing a first embodiment of the present invention, wherein
Fig. 1 is a perspective view which shows an endoscope hood mounted on the distal end portion of an endoscope insertion portion;
Fig. 2 is a longitudinal cross-sectional view along the axis of the insertion portion, which shows the endoscope hood and the endoscope insertion portion taken along line II-II in Fig. 1;
Figs. 3 and 4 are diagrams for describing a second embodiment of the present invention, wherein

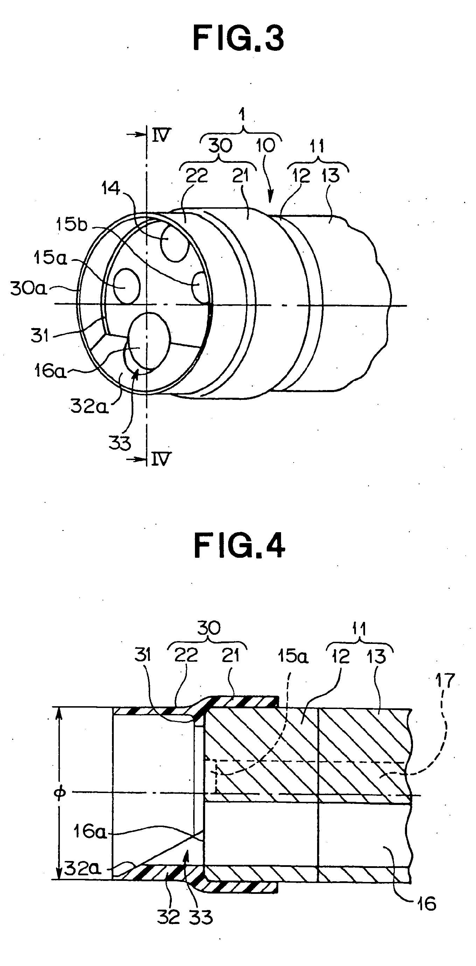

Fig. 3 is a perspective view which shows an endoscope hood mounted on the distal end portion of an endoscope insertion portion;
Fig. 4 is a longitudinal cross-sectional view along the axis of the insertion portion, which shows the endoscope hood and the endoscope insertion portion taken along line IV-IV in Fig. 3;

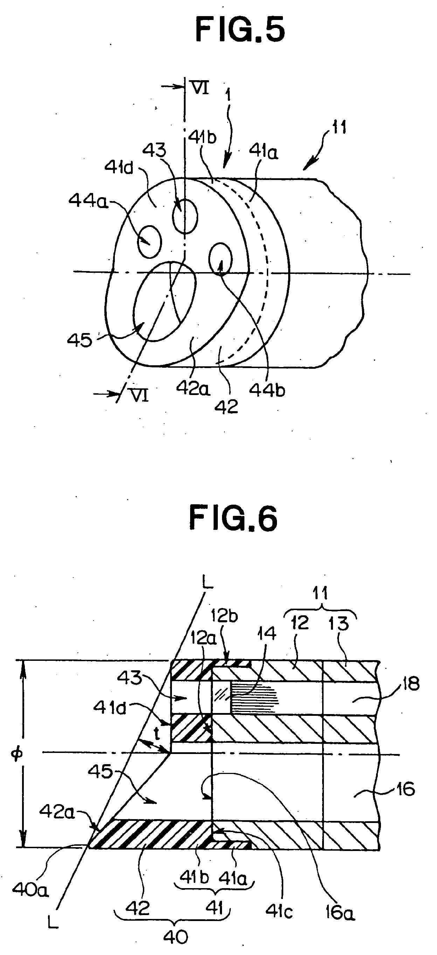

Figs. 5 and 6 are diagrams for describing a third embodiment of the present invention, wherein
Fig. 5 is a perspective view which shows an endoscope hood mounted on the distal end portion of an endoscope insertion portion; and
Fig. 6 is a longitudinal cross-sectional view along the axis of the insertion portion, which shows the endoscope hood and the endoscope insertion portion taken along line VI-VI in Fig. 5.

### DETAILED DESCRIPTION OF THE PREFERRED EMBODIMENTS

Description will be made below regarding embodiments according to the present invention with reference to the drawings.

Now, description will be made regarding a first embodiment according to the present invention with reference to Figs. 1 and 2.

An endoscope device 1 according to the present invention comprises an endoscope 10 and an endoscope hood 20. The endoscope 10 includes a flexible insertion portion 11 which is to be inserted into the body cavity. The insertion portion 11 comprises: a distal end portion 12 formed of a rigid material; a bending portion 13 formed of muldistal endle joint members (not shown) connected one to another; and a flexible tube portion (not shown) having suitable flexibility, in that order from the distal end, for example.

The distal end portion 12 forming the insertion portion 11 includes: an observation window 14; two illumination ports 15a and 15b, for example; and a channel opening 16a (which will be abbreviate to "opening" hereafter) forming a forceps channel 16, on the distal end surface 12a.

Furthermore, an objective lens (not shown) is provided behind the observation window 14 for forming an observation optical system. Furthermore, the image pickup surface of an image pickup device is disposed at the focal position of the objective lens, for example. An image pickup apparatus (not shown) having the image pickup device includes a signal cable (not shown) extending from the proximal end thereof for transmitting control signals and electric signals. The signal cable passes through the insertion portion 11, and is connected to a camera control unit (not shown) forming the endoscope device 1.

On the other hand, the output end surfaces of a light guide optical fiber bundle 17 is provided to the proximal ends of the illumination ports 15a and 15b. On the other hand, the input end of the light guide optical fiber bundle 17 is disposed so as to face an illumination lamp provided to a light source device (not shown) forming the endoscope device 1. Illumination light transmitted through the light guide optical fiber bundle 17 passes through the illumination ports 15a and 15b, and is cast onto the tissue portion which is to be observed.

The opening 16a is included for introducing a treatment tool for various kinds of treatment or the like. The forceps channel 16 according to the present embodiment is used as a suctioning channel forming a tube channel having a suctioning function, as well as being used as a channel for inserting a treatment tool. Note that an arrangement may be made wherein the suctioning channel is also used as the air/water feed channel. Furthermore, the endoscope may include the suctioning channel and the forceps channel as separate units. In this case, the suctioning channel may be formed as a separate unit which is detachably mounted to the insertion portion.

The endoscope hood 20 is detachably mounted on the outer surface of the distal end portion 12. The endoscope hood 20 is mounted on the endoscope 10 such that the distal end surface 20a of the endoscope hood 20 protrudes from the distal end surface 12a of the distal end portion 12 by a predetermined length.

The endoscope hood 20 is formed of a flexible tube with a small thickness generally in the shape of a tube. The endoscope hood 20 comprises: a mounting portion 21; and a hood portion 22 protruding from the distal end surface 12a of the distal end portion 12. The endoscope hood 20 is elastically mounted on the outer surface of the distal end portion 12 through the elastic mounting portion 21, whereby the endoscope 10 and the endoscope hood 20 form a single unit.

On the other hand, the hood portion 22 protrudes from the distal end surface 12a of the distal end portion 12 toward the endoscope-insertion direction, i.e., toward observing viewing direction of the endoscope 10. The hood portion 22 of the endoscope hood 20 is formed with a length such that the hood portion 22 protrudes from the distal end surface 12a of the distal end portion 12 toward the observing viewing direction by a predetermined length, giving consideration to observation of the tissue.

The hood portion 22 is formed with an outer diameter (φ) equal to or smaller than the outer diameter of the distal end portion 12. Accordingly, the hood portion 22 is formed with an outer diameter smaller than the outer diameter of the mounting portion 21.

The inner surface of endoscope hood 20 formed of the mounting portion 21 and the hood portion 22 includes a ridge 23 along a part of the inner circumference thereof or over the entire inner circumference thereof at a predetermined portion along the axial direction. The ridge 23 is formed with a predetermined width in the longitudinal direction and a predetermined height. Note that the height of the ridge 23 is determined such that the observation window 14, and the illumination ports 15a and 15b, included in the distal end surface 12a, are not obstructed by the endoscope hood 20 mounted on the endoscope 10.

The side wall of the ridge 23 on the mounting-portion side is formed as a surface for being in contact with the distal end surface 12a and the outer surface of the distal end portion 12. Accordingly, the physician mounts the endoscope hood 20 such that the side wall of the ridge 23 on the mounting-portion side is in contact with the distal end surface 12a. In this case, the hood portion 22 protrudes from the distal end surface 12a by a predetermined length.

On the other hand, the side wall of the ridge 23 on the distal end side is formed in the shape of a curved surface or an inclined surface, which will be referred to as a slope 24 hereafter. The slope 24 is formed such that the inner diameter of the ridge 23 gradually becomes greater the closer to the distal end of the hood portion 22, whereby the side wall of the ridge on the distal end side is formed in the shape of a slope, i.e., the side wall of the ridge on the distal end side is formed such that the height is changed along the longitudinal direction. In other words, the ridge 23 gradually becomes smaller the closer to the distal end of the hood portion 22. This improves mechanical strength of the hood portion 22 while maintaining the field of view.

The ridge 23 thus formed with a predetermined width and height includes a notch 25 for facilitating lead-out of a treatment tool through the opening 16a. The notch is formed in a shape such that the treatment tool can be smoothly introduced from the opening 16a.

As described above, formation of the notch 25 on the ridge 23 allows a layout wherein the opening 16a is formed on the perimeter of the distal end surface 12a. This allows a layout wherein the observation window 14 is disposed around the middle portion of the distal end surface 12a, thereby preventing shading.

Now, description will be made regarding mounting of the endoscope hood 20 having such a configuration onto the distal end portion 12, and operations of the mounted endoscope hood 20.

First, the physician fits the mounting portion 21 of the endoscope hood 20 to the distal end portion 12 of the insertion portion 11. Then, the physician pushes the mounting portion 21 toward the bending portion 13 until the side wall of the ridge 23 on the mounting-portion side formed on the inner wall, comes in contact with the distal end surface 12a of the distal end portion 12. Subsequently, the physician adjusts the position of the endoscope hood 20 such that the notch 25 is positioned in front of the opening 16a. Thus, the endoscope hood 20 is suitably mounted on the distal end portion 12.

With the endoscope having the endoscope hood mounted thereon as described above, the distance between the distal end surface 20a of the hood portion 22 and the observation window 14 provided to the distal end surface 12a is determined to be a suitable predetermined distance for endoscopic observation. Furthermore, with the present embodiment, the opening 16a is not obstructed by the ridge 23 forming the inner wall of the endoscope hood 20. Furthermore, with the present embodiment, the observation window 14, and the illumination ports 15a and 15b, formed on the distal end surface 12a, are disposed around the middle portion as compared with the edge of the ridge 23, and accordingly, the window and ports are not obstructed by the endoscope hood 20 mounted on the endoscope 10.

Accordingly, illumination light output from the illumination ports 15a and 15b is cast onto the tissue which is to be observed without being shielded due to the ridge 23. Furthermore, with such a configuration, the physician can observe the tissue portion, which is to be observed, through the observation window 14 under optimum conditions without problems such as shading or the like. Furthermore, the endoscope hood 20 having such a configuration wherein the ridge 23 is disposed along the inner surface of the endoscope hood 20 with the distal end of the ridge positioned at the midway in the axial direction of the inner surface so that the hood portion 22 allows the physician to perform suctioning of waste or the like, without a problem that the opening 16a is closed off by the tissue, while suppressing suctioning of mucous tissue, as well as allowing the physician to smoothly introduce a treatment tool to the intended portion through the opening 16a and the notch 25.

Note that an arrangement may be made wherein the ridge 23 is formed with a greater height such that the edge of the ridge 23 is positioned closer to the middle portion of the endoscope hood 20 than the observation window 14, and the illumination ports 15a and 15b, and corresponding notches are formed in front of these ports in the same way as with the aforementioned notch 25.

As described above, the endoscope hood 20 comprises the mounting portion and the hood portion formed with a smaller diameter than with the distal end portion, thereby maintaining the outer diameter of the distal end portion of the endoscope with the endoscope hood equal to or smaller than the outer diameter of that without the endoscope hood. This maintains ease of insertion of the insertion portion with the endoscope hood mounted on the distal end portion thereof.

Furthermore, the endoscope hood 20 includes the ridge at a predetermined position along the inner surface thereof with the distal end of the ridge positioned at the midway in the axial direction of the inner surface. Accordingly, at the time of mounting the endoscope hood 20, the physician mounts the endoscope hood 20 such that the side wall of the ridge on the proximal-end side is in contact with the distal end surface of the distal end portion, whereby the endoscope hood 20 is easily mounted on the endoscope 10 in a surer manner. Furthermore, this allows the physician to observe the tissue with a suitable distance between: the observation optical system; and the tissue, which is to be observed; the distance being determined by the side wall of the ridge on the proximal-end-side surface in contact with the tissue which is to be observed, thereby obtaining clear endoscopic images through the observation optical system.

Furthermore, with the present embodiment, the side wall of the ridge is formed in the shape of a slope on the distal end side, thereby improving the mechanical strength of the mounting portion and the hood portion which are formed with small thickness. This allows design of the hood portion with a smaller diameter than with the mounting portion while maintaining the mechanical strength of the hood portion.

Furthermore, with the present embodiment, the endoscope hood is designed such that the slope of the ridge is formed giving consideration to the field of view of the observation optical system, specifically, the endoscope hood is designed such that the field of view of the observation optical system is not obstructed by the slope, thereby enabling observation without shading.

Furthermore, with the present embodiment, the ridge includes the notch which communicates with the opening of the tube channel having a suctioning function; the front end of the notch being positioned on the proximal end side as compared with the distal end surface of the endoscope hood. This allows the physician to perform suctioning of waste within the body cavity with the distal end surface of the endoscope hood in contact with the tissue while preventing suctioning of mucous tissue without a problem that the opening is closed off by the tissue. Furthermore, with the present embodiment, the tube channel is included within the insertion portion, this allows design wherein the hood portion is formed with a small diameter, thereby reducing the diameter of the distal end portion with the endoscope hood mounted thereon.

Note that description has been made regarding an arrangement wherein the slope 24 on the side of the hood portion 22 is formed as a surface curved in a direction with reference to Fig. 2, a further arrangement may be made wherein the slope 24 is formed into a spherical surface curved in two directions.

With an arrangement wherein the slope 24 is formed as a plane surface instead of a curved surface, a further arrangement may be made wherein the slope 24 is formed into a concaved surface, so that the slope 24 is in contact with the tissue with a gap between the mucous tissue and the recessed surface structure during observation of the tissue, thereby preventing suctioning of mucous tissue.

While description has been made regarding an arrangement wherein the endoscope hood 20 includes the mounting portion 21 formed of an elastic material, and the endoscope hood 20 is mounted onto the distal end portion 12 of the insertion portion 11 through the mounting portion 21, an arrangement may be made wherein the endoscope hood 20 which comprises: the hood portion 22; the slope 24; the notch 25; and the like, is integrally formed on the distal end portion 12.

Furthermore, an arrangement may be made wherein the forceps channel and a channel having a suctioning function are formed as separate channels. In this case, an arrangement may be made wherein the slope 24 includes another opening at the slanted portion corresponding to the opening of the channel having the suctioning function, as well.

Furthermore, an arrangement may be made wherein the ridge is formed with a height such that the edge of the ridge is closer to the middle portion of the endoscope hood 20 than the any portion of the opening of the tube channel having the suctioning function, and the slope of the ridge includes an opening corresponding to the opening of the tube channel, instead of the notch.

Next, description will be made regarding a second embodiment according to the present invention with reference to Figs. 3 and 4.

An endoscope hood 30 according to the present embodiment has a different structure of the inner surface from that of the endoscope hood 20 described above. Specifically, the endoscope hood 30 includes a ridge 31 and a slope 32 on the inner surface thereof. Note that the same components as with the aforementioned first embodiment are denoted by the same reference numerals, and description thereof will be omitted. Note that the slope 32 may serve as the side wall of the ridge on the distal end side, which is formed such that the height is changed along the longitudinal direction.

The ridge 31 is formed on the inner surface along a part of the inner circumference or over the entire circumference at a predetermined portion along the axial direction with a predetermined height and width. The side wall of the ridge 31 on the proximal-end-side surface is formed as a surface for being in contact with the distal end surface 12a of the distal end portion 12.

The slope 32 is disposed such that the distal end thereof is positioned in front of the ridge 31 along the axial direction of the insertion portion 11, i.e., along the forward viewing direction, and has a slope surface 32a with a predetermined slope angle. As can be understood from the cross-sectional view of the slope 32 shown in Fig. 4, the slope 32 is formed with the maximum thickness at the position of the ridge 31, and is formed with the minimum thickness around the distal end surface 30a. Note that the slope angle of the slope 32 is determined to be equal to or greater than the observing viewing angle of the observation window 14 giving consideration to observation through the observation window 14.

The slope 32 includes a notch 33 which smoothly communicates with the opening 16a provided on the distal end portion 12 of the insertion portion 11. The notch 33 is formed on the slope surface 32a such that the front end thereof along the viewing direction is positioned closer to the proximal end side than the distal end surface 30a of the hood 22.

With such a configuration, at the time of mounting of the endoscope hood, the physician mounts the endoscope hood on the endoscope such that the side wall of the ridge on the proximal-end-side surface formed on the inner surface of the endoscope hood is in contact with the distal end surface of the distal end portion, thereby facilitating mounting of the endoscope hood in a surer manner.

Furthermore, such a configuration wherein the slope is formed on the inner surface of the endoscope hood ahead of the ridge along the direction toward the distal end of the endoscope hood improves the mechanical strength of the hood portion formed with a small thickness.

Furthermore, such a configuration wherein the slope is formed with a slope angle equal to or greater than the observing viewing angle prevents the problem of shading.

Furthermore, such a configuration, wherein the notch which communicates with the opening having a suctioning function is formed such that the distal end thereof is positioned closer to the proximal end side than the distal end surface of the endoscope hood, allows the physician to perform suctioning of waste within the body cavity with the distal end surface of the endoscope hood in contact with the tissue while preventing suctioning of the mucous tissue without a problem that the opening is closed off by the tissue.

Other functions and advantages of the present embodiment are the same as with the first embodiment described above.

Note that while description has been made regarding an arrangement wherein the slope 32 is formed in the hood portion 22 of the endoscope hood 30 with the slope surface 32a as a flat surface with reference to Figs. 3 and 4, an arrangement may be made wherein the slope surface 32a is formed as a recessed surface. With such a configuration wherein the slope surface 32a is formed as a recessed surface, this configuration allows the physician to perform suctioning of waste with the slope surface 32a in contact with the tissue with a gap therebetween, thereby preventing suctioning of mucous tissue in a surer manner.

While description has been made in the present embodiment regarding an arrangement wherein the endoscope hood 30 including the mounting portion 21 is mounted on the distal end portion 12 of the insertion portion 11 with the mounting portion 21, an arrangement may be made wherein the distal end portion 12 and the endoscope hood 30 comprising: the hood portion 22; the slope 32; the notch 33; and so forth, are integrally formed.

Next, description will be made regarding a third embodiment of the present invention with reference to Figs. 5 and 6.

An endoscope hood 40 according to the present embodiment is detachably mounted on a hood mounting step (which will be abbreviated to "step" hereafter) 12b formed along the outer circumference of the distal end portion 12 of the insertion portion 11. Note that the same components as with the aforementioned first embodiment are denoted by the same reference numerals, and description thereof will be omitted.

The distal end surface 12a of the distal end portion 12 of the endoscope 10 includes: the opening 16a of the forceps channel 16; the observation window 14; and multiple illumination ports (not shown), for example. With the present embodiment, the distal end surface of the image-guide optical-fiber bundle 18 is provided to the proximal end of the observation window 14. Furthermore, the step 12b is formed on the outer circumference of the distal end portion 12 on the distal end side thereof.

The endoscope hood 40 comprises: a mounting portion 41 for being mounted on the step 12b; and a hood portion 42 having a front end 40a serving as a protrusion protruding from the distal end surface 12a by a predetermined length. The mounting portion 41 is formed in the shape of a cylinder, and comprises a ring-shaped portion 41a and a cylindrical portion 41b. The endoscope hood 40 is mounted on the endoscope with the bottom 41c of the mounting portion 41 in contact with the distal end surface 12a. The ring-shaped portion 41a is elastically fixed on the step 12b. The hood portion 42 is formed solid, i.e., not hollow, and has an inclined surface 42a formed with a predetermined inclined angle as to a vertical surface 41d orthogonal to the central axis of the mounting portion 41.

With the present embodiment, the mounting portion 41 and the hood portion 42 are formed with the outer circumference parallel to the central axis thereof. Note that the endoscope hood 40 is formed with an outer diameter (φ) equal to or smaller than the outer diameter of the distal end portion 12 of the insertion portion 11.

The vertical surface 41d of the mounting portion 41 includes an observation through-hole 43 which communicates with the observation window 14 provided to the distal end surface 12a of the distal end portion 12, and illumination through holes 44a and 44b which communicate with corresponding illumination ports (not shown). On the other hand, the hood portion 42 includes a forceps through hole 45 which communicates the opening 16a. The forceps through hole 45 is formed with a diameter equal to or greater than the diameter of the opening 16a. Note that no notch is formed on the inclined surface 42a which communicates with the distal end of the forceps through hole 45.

With the endoscope having the endoscope hood 40 mounted on the distal end portion 12 thereof, at the time of the physician inserting the endoscope into the body cavity, the distal end of the mounting portion 41 of the endoscope hood 40 and the distal end of the hood portion 42 thereof come in contact with the intended tissue portion with a space denoted by reference character t surrounded by the L-L line shown in the drawing, the vertical surface 41d, and the inclined surface 42a.

Note that while description has been made in the present embodiment regarding an arrangement wherein the observation through-hole 43 is formed as a straight hole as shown in Fig. 6, the observation through-hole 43 and the illumination through holes 44a and 44b may be formed as taper holes, or may be formed such that at least a part of the side wall of the hole is formed slanting so as to expand the hole area along the observation viewing direction.

With such an endoscope hood having a configuration wherein the mounting portion provided to the endoscope hood is fit to the step formed on the distal end portion of the endoscope, the physician mounts the endoscope hood on the endoscope such that the bottom of the endoscope hood is in contact with the distal end surface of the distal end portion of the endoscope, thereby facilitating mounting of the endoscope hood onto the endoscope in a surer manner.

Furthermore, with such a configuration wherein an inclined surface is formed on the hood portion of the endoscope hood for being mounted on the distal end portion of the endoscope, the hood portion is formed such that the cross-sectional area of the hood portion becomes smaller the closer to the distal end thereof, and accordingly, the hood portion having the inclined surface serves as a guide portion at the time of insertion of the insertion portion of the endoscope, thereby facilitating insertion of the insertion portion of the endoscope with the endoscope hood mounted thereon.

Furthermore, such a configuration wherein the hood portion includes the forceps through hole formed on the inclined surface thereof allows the physician to perform suctioning of waste within the body cavity with the front end of the endoscope hood in contact with the tissue while preventing suctioning of the mucous tissue without the problem of the opening being closed off by tissue.

Furthermore, with such a configuration, at the time of the physician inserting the endoscope into the body cavity, and operating the endoscope so as to bend the bending portion thereof such that a part of the mounting portion and a part of the hood portion come into contact with the tissue, a space denoted by reference character t is formed surrounded by: the line L-L shown in the drawing; the vertical surface 41d; and the inclined surface 42a; thereby preventing in a surer manner the problem of the distal end of the forceps through hole being closed off by the tissue.

Other functions and advantages are the same as with the aforementioned embodiments.

While description has been made in the present embodiment regarding an arrangement wherein the inclined surface 42a on the hood portion 42 of the endoscope hood 40 is formed as a flat surface with reference to Figs. 5 and 6, the inclined surface 42a may be formed as a recessed surface. With such a configuration wherein the inclined surface 42a is formed as a recessed surface, at the time of the physician inserting the distal end portion of the endoscope into the body cavity with the inclined surface 42a in contact with the tissue, a space is formed between the recessed surface and the mucous tissue, thereby preventing the problem of suctioning of the mucous tissue.

Also, while description has been made in the present embodiment regarding an arrangement wherein the mounting portion 21 of the endoscope hood 40 includes the ring-shaped portion 41a so as to be fixed to the step 12b provided to the distal end portion 12 of the insertion portion 11, an arrangement may be made wherein at least the hood portion 42 which is a part of the endoscope hood 40 is integrally formed on the distal end portion 12, instead of formation of the step 12b. Furthermore, an arrangement may be made wherein the inclined surface 42a of the hood portion 42 is formed with an inclined angle equal to or greater than the observing viewing angle, thereby preventing of a problem of shading.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An endoscope hood comprising:
a hood portion provided to an insertion portion of an endoscope for being inserted into the body cavity, which includes a tube channel having a suctioning function, so as to extend in the forward viewing direction ahead of the distal end surface of an observation optical member for observing the tissue within the body cavity; and
a ridge formed on at least a part of the hood portion, which has a slope formed such that the cross-sectional thickness thereof becomes smaller toward the forward viewing direction;
wherein at least a part of the opening of the tube channel having the suctioning function is formed on the slope;
and wherein the opening at the forward viewing direction is formed so as to be positioned closer to the observation window than the distal end of the hood portion formed near the tube channel.

2. An endoscope hood according to Claim 1, wherein the slope is formed of a curved surface.

3. An endoscope hood according to Claim 1, wherein the slope is formed of an inclined surface.

4. An endoscope hood according to Claim 1, wherein the endoscope hood includes at least a part of a tubular portion;
and wherein the tubular portion is formed with the generally the same diameter as with the distal end portion of the insertion portion.

5. An endoscope hood according to Claim 1, further including a mounting portion for being detachably mounted on the distal end portion of the insertion portion, wherein the ridge includes a communicating portion which allows the opening formed on the slope to communicate with the opening of the tube channel formed on the distal end of the insertion portion of the endoscope.

6. An endoscope hood according to Claim 4, further including a mounting portion for being detachably mounted on the distal end portion of the insertion portion, wherein the ridge includes a communicating portion which allows the opening formed on the slope to communicate with the opening of the tube channel formed on the distal end of the insertion portion of the endoscope.

7. An endoscope hood according to Claim 1, wherein the hood portion is integrally formed on the insertion portion of the endoscope.

8. An endoscope hood according to Claim 4, wherein the hood portion is integrally formed on the insertion portion of the endoscope.

9. An endoscope hood comprising:
a hood portion provided to an insertion portion of an endoscope for being inserted into the body cavity, which extends ahead of the distal end surface of an observing optical member for observing the tissue within the body cavity in the forward viewing direction with the cross-sectional thickness thereof becoming smaller toward the forward viewing direction; and
an opening of the tube channel having a suctioning function, wherein at least a part thereof is formed on the hood portion with the cross-sectional thickness thereof becoming smaller toward the forward viewing direction.

10. An endoscope hood comprising:
a hood portion provided to an insertion portion of an endoscope for being inserted into the body cavity, which extends ahead of the distal end surface of an observing optical member for observing the tissue within the body cavity in the forward viewing direction; and
an opening of the tube channel having a suctioning function wherein at least a part thereof is formed on the hood portion;
wherein the distal end of the opening of the tube channel on the most-forward side is positioned behind the distal end of the hood portion in the forward viewing direction.
